# EUROPEAN PATENT APPLICATION

(11) **EP 2 962 631 A1**
(43) Date of publication of application: **06.01.2016**
(21) Application number: 15174658.3
(22) Date of filing: 30.06.2015
(51) Int. Cl.: A61B 5/00

(54) **PHOTOACOUSTIC IMAGE PRODUCTION DEVICE**

(30) Priority: 02.07.2014 JP 2014136677
(71) Applicant: Funai Electric Co., Ltd., Daito-shi, Osaka 574-0013 (JP)
(72) Inventor: Kitagawa, Kazuo, Daito-shi, Osaka 574-0013 (JP)
(74) Representative: Global IP Europe Patentanwaltskanzlei

(57) **Abstract**

A photoacoustic image production device includes a light source configured to emit light, a light source driver configured to cause the light source to emit the light, an ultrasonic wave detector configured to receive an ultrasonic wave and produces an ultrasonic wave detection signal, an image signal production component configured to produce an ultrasound image based on the ultrasonic wave detection signal, and a controller configured to control the light source driver to stop operation of a booster circuit during a first reception period in which the ultrasonic wave detector receives the ultrasonic wave produced by the light emitted from the light source.

## Description

### CROSS-REFERENCE TO RELATED APPLICATIONS

This application claims priority to Japanese Patent Application No. 2014-136677 filed on July 2, 2014. The entire disclosure of Japanese Patent Application No. 2014-136677 is hereby incorporated herein by reference.

### BACKGROUND

### Field of the Invention

The present invention generally relates to a photoacoustic image production device. More specifically, the present invention relates to a photoacoustic image production device with a light source that emits light toward a test object.

### Background Information

There are conventional photoacoustic image production devices that produce an ultrasound image of a test object by shining measurement light at a test object (a human body, etc.) and detecting acoustic waves produced by a photoacoustic effect within the test object. With this device, the measurement light is emitted from an ultrasonic probe side toward the inside of the test object in a state in which the ultrasonic probe is in contact with the test object, and the acoustic waves (ultrasonic waves) generated within the test object as a result of this measurement light are detected by an ultrasonic wave detector inside the ultrasonic probe.

Light tends to be attenuated within a test object, and measurement light with a high light output corresponding to the measurement depth is emitted from the light source to ensure a large measurement depth. Therefore, a large laser device (solid state laser device, etc.) is generally used as the light source of this device.

Because a laser device such as this is large in size, it is provided separately from the ultrasonic probe that is brought into contact with the test object, and the laser pulses generated by the laser device are supplied from the laser device to the ultrasonic probe through an optical fiber (see Japanese Laid-Open Patent Application Publication No. 2012-187389 (Patent Literature 1), for example).

### SUMMARY

However, incorporating a laser device ends up making the entire device larger, the device becomes more expensive, and this is a hindrance to installing it in a typical medical facility. In view of this, the inventors have been proposed a photoacoustic image production device in which a compact light emitting diode (LED) chip, a semiconductor laser chip, or another such light emitting element is used instead of a laser device as a light source. Using this compact light emitting element allows the light source to be disposed in the ultrasonic probe so that it is inside the ultrasonic probe housing or near the ultrasonic probe housing, so the overall device can be smaller and it can be manufactured at a much lower price.

When an LED chip is used, for example, the light output per chip is relatively small, so a plurality of LED chips has to be used to ensure the required amount of light output. Connecting a large number of LED chips in parallel results in a very large quantity of wires, so an LED circuit composed of a serial connection circuit of a plurality of LED chips (or a plurality of serial connection circuits connected in parallel) is incorporated into an ultrasonic probe in an effort to avoid an increase in the size and weight of the ultrasonic probe. Also, since there is variance in the forward voltage Vf from one LED chip to the next, when LED chips are connected in series, there ends up being variance in the brightness of the LED chips. To reduce this brightness variation, a resistor has to be inserted into the LED chips, etc., and this makes the circuit larger. Therefore, to avoid this problem, it has been discovered that an LED circuit composed of a serial connection circuit of a plurality of LED chips is employed.

To this LED circuit is applied a voltage equal to the forward voltage Vf of the LED chip (such as 1.4 to 6 V) multiplied by the number of LED chips. For example, with an LED circuit in which 70 LED chips with the forward voltage Vf of 3 V are serially connected, a voltage of at least 210 V (= 3 V x 70) is applied to both ends of the circuit. And to produce such a high voltage, a booster circuit such as a DC-DC converter is required. The booster circuit includes an internal switching element, and can operate this switching element at high speed to boost the power supply voltage to the above-mentioned high voltage level.

On the other hand, it has also been discovered that when the switching element is switched in the booster circuit, this can generate electromagnetic noise. The inventors have also discovered a new problem, in that this electromagnetic noise can end up being added to the ultrasonic wave detection signal produced by the ultrasonic probe that has received the ultrasonic waves, disturbance can occur in the ultrasound image produced as a result, and sharpness can end up being lost.

One object is to provide a photoacoustic image production device with which electromagnetic noise that is produced by an operation of a booster circuit that produces a drive voltage of a light source can be prevented from being mixed into an ultrasonic wave detection signal, and sharpness of an ultrasound image that is produced can be achieved.

In view of the state of the known technology and in accordance with a preferred embodiment, a photoacoustic image production device is provided that comprises a light source configured to emit light, a light source driver configured to cause the light source to emit the light, an ultrasonic wave detector configured to receive an ultrasonic wave and produce an ultrasonic wave detection signal, an image signal production component configured to produce an ultrasound image based on the ultrasonic wave detection signal, and a controller configured to control the light source driver to stop operation of a booster circuit during a first reception period in which the ultrasonic wave detector receives the ultrasonic wave produced by the light emitted from the light source.

In accordance with another preferred embodiment according to the photoacoustic image production device mentioned above, the first reception period is a period from emission of the light by the light source until reception of the ultrasonic wave produced at a specific measurement depth in a test object by the light.

In accordance with another preferred embodiment according to any one of the photoacoustic image production device mentioned above, the light source includes light emitting diodes, semiconductor laser chips or organic light emitting diodes that are serially connected to each other.

In accordance with another preferred embodiment according to any one of the photoacoustic image production device mentioned above, the booster circuit is a booster circuit for a light source driver.

In accordance with another preferred embodiment according to any one of the photoacoustic image production device mentioned above, the photoacoustic image production device further comprises an ultrasonic wave generator configured to output an ultrasonic wave in a test object, the ultrasonic wave detector being configured to receive a reflected wave produced when the ultrasonic wave outputted from the ultrasonic wave generator is reflected inside the test object, and configured to produce an ultrasonic wave detection signal based on the received reflected wave.

In accordance with another preferred embodiment according to any one of the photoacoustic image production device mentioned above, the controller is configured to control output of the ultrasonic wave by the ultrasonic wave generator, and configured to control the light source driver to stop the operation of the booster circuit during a second reception period in which the ultrasonic wave detector receives the reflected wave of the ultrasonic wave outputted from the ultrasonic wave generator.

In accordance with another preferred embodiment according to any one of the photoacoustic image production device mentioned above, the second reception period is a period from the output of ultrasonic wave by the ultrasonic wave generator until reception of the reflected wave produced by reflecting the ultrasonic wave at a specific measurement depth within the test object.

In accordance with another preferred embodiment according to any one of the photoacoustic image production device mentioned above, the controller is configured to control the light source driver to drive the booster circuit for a specific drive period at specific repetition cycle and to emit the light from the light source for a specific emission period, and configured to control the ultrasonic wave generator to output the ultrasonic wave from the ultrasonic wave generator for a specific output period, the specific repetition cycle is at least a period obtained by adding the first reception period, the second reception period, and the drive period of the booster circuit.

In accordance with another preferred embodiment according to any one of the photoacoustic image production device mentioned above, the ultrasonic wave detector and the ultrasonic wave generator commonly include a piezoelectric element.

In accordance with another preferred embodiment according to any one of the photoacoustic image production device mentioned above, the light source is disposed near the ultrasonic wave detector.

In accordance with another preferred embodiment according to any one of the photoacoustic image production device mentioned above, the light source is disposed near the booster circuit.

In accordance with another preferred embodiment according to any one of the photoacoustic image production device mentioned above, the light source driver is configured to drive the light source with a specific drive voltage, and the controller is configured to control the light source driver to increase a charging voltage of the booster circuit to the drive voltage after emission of the light by the light source.

In accordance with another preferred embodiment according to any one of the photoacoustic image production device mentioned above, the controller is configured to control the light source driver to increase the charging voltage of the booster circuit to the drive voltage after elapsing the first reception period.

In accordance with another preferred embodiment according to any one of the photoacoustic image production device mentioned above, the light source driver is configured to drive the light source with a specific drive voltage, and the controller is configured to control the light source driver to increase a charging voltage of the booster circuit to the drive voltage after the output of the ultrasonic wave by the ultrasonic wave generator.

In accordance with another preferred embodiment according to any one of the photoacoustic image production device mentioned above, the controller is configured to control the light source driver to increase the charging voltage of the booster circuit to the drive voltage after elapsing the second reception period.

Also other objects, features, aspects and advantages of the present disclosure will become apparent to those skilled in the art from the following detailed description, which, taken in conjunction with the annexed drawings, discloses one embodiment of the photoacoustic image production device.

### BRIEF DESCRIPTION OF THE DRAWINGS

Referring now to the attached drawings which form a part of this original disclosure:
FIG. 1 is an electrical block diagram of a photoacoustic image production device pertaining to a first embodiment;
FIG. 2 is a simplified perspective view of an ultrasonic probe of the photoacoustic image production device pertaining to the first embodiment;
FIG. 3 is an electrical block diagram of an inside of the ultrasonic probe of the photoacoustic image production device pertaining to the first embodiment;
FIG. 4 is a diagram illustrating a mechanism by which acoustic waves are generated by a photoacoustic effect;
FIG. 5 is a flowchart of measurement processing performed by the photoacoustic image production device pertaining to the first embodiment;
FIG. 6 is a timing chart of measurement operation of the photoacoustic image production device pertaining to the first embodiment;
FIG. 7 is an electrical block diagram of a photoacoustic image production device pertaining to a second embodiment;
FIG. 8 is a diagram illustrating a mechanism by which reflected waves are generated;
FIG. 9 is a flowchart of measurement processing performed by the photoacoustic image production device pertaining to the second embodiment;
FIG. 10 is a timing chart of measurement operation of the photoacoustic image production device pertaining to the second embodiment; and
FIG. 11 is a simplified perspective view of an ultrasonic probe of a photoacoustic image production device pertaining to a modification example.

### DETAILED DESCRIPTION OF EMBODIMENTS

Selected embodiments will now be explained with reference to the drawings. It will be apparent to those skilled in the art from this disclosure that the following descriptions of the embodiments are provided for illustration only and not for the purpose of limiting the invention as defined by the appended claims and their equivalents.

### FIRST EMBODIMENT

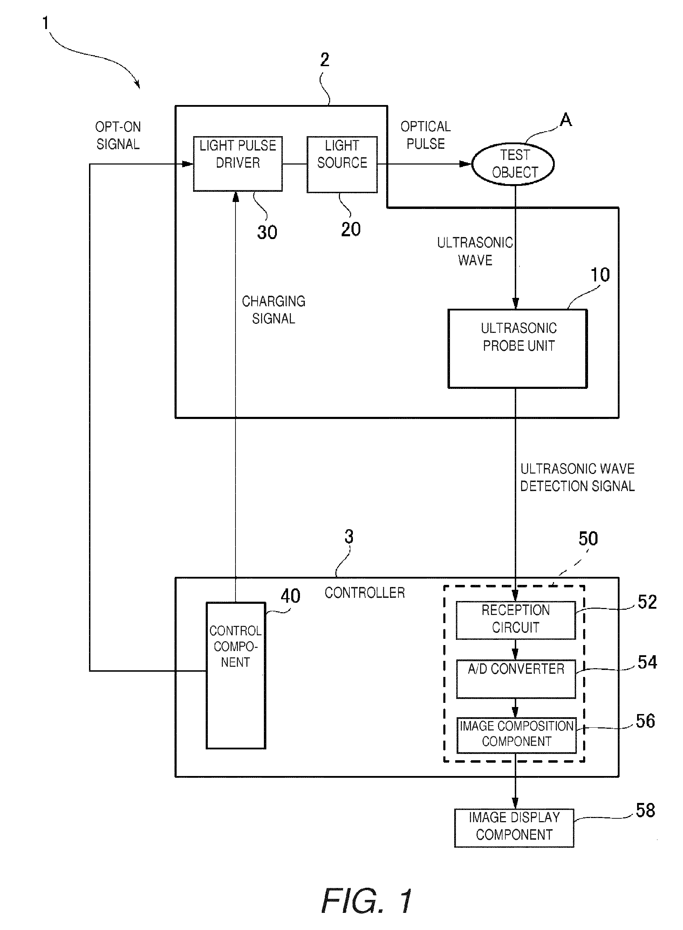

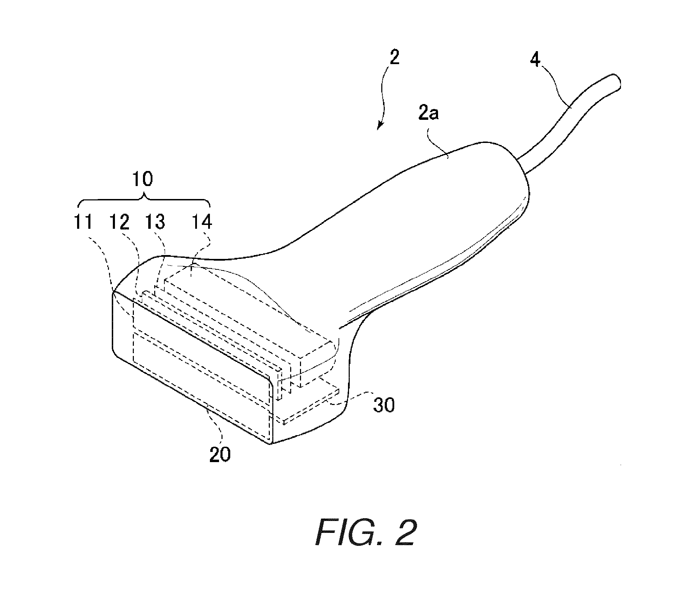

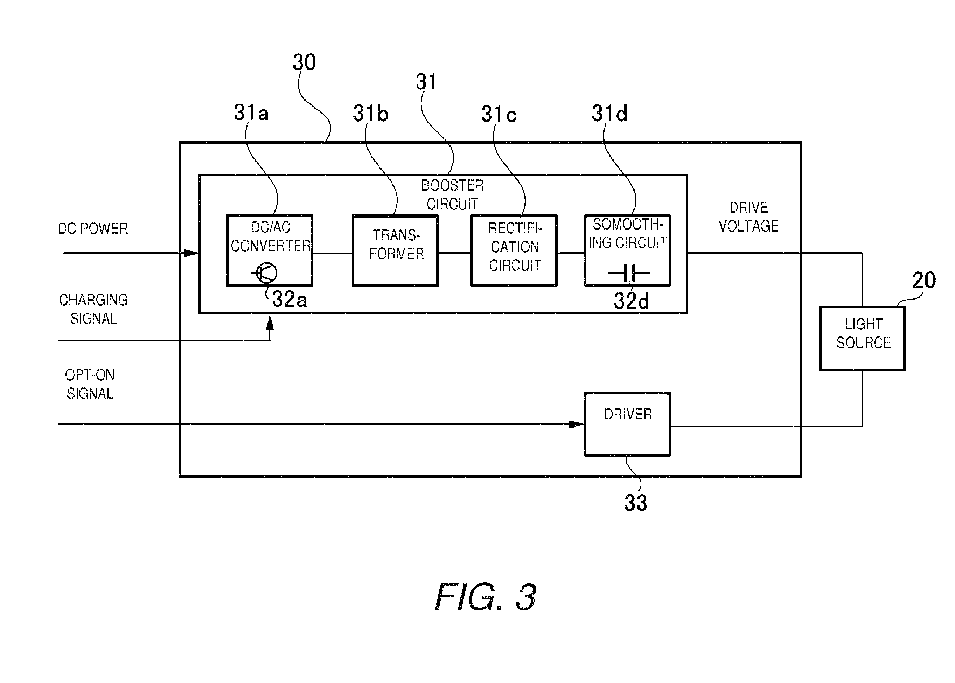

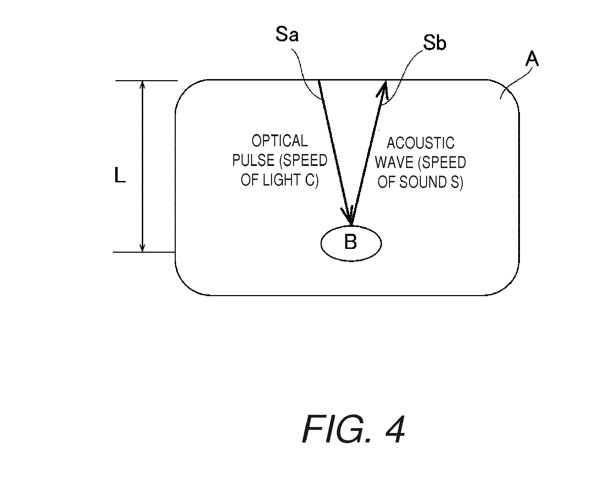

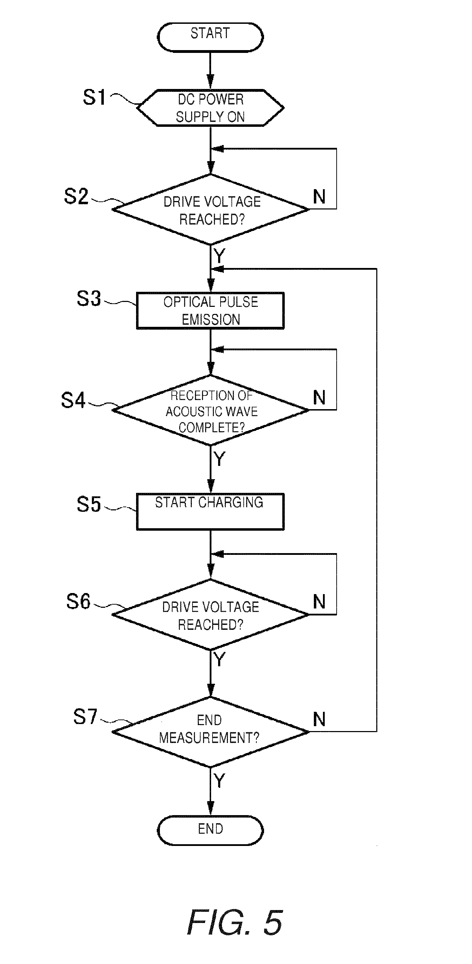

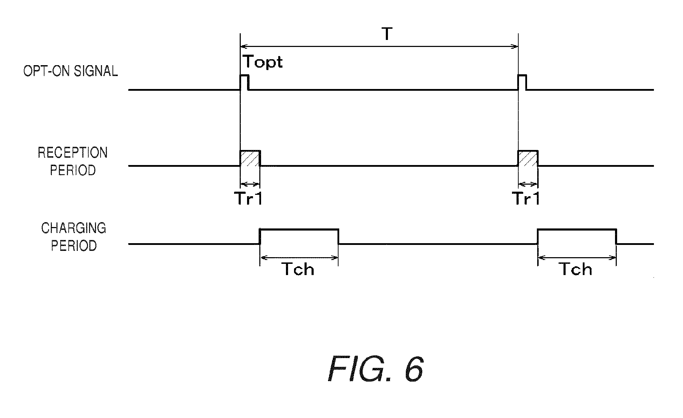

Referring to FIGS. 1 to 6, a photoacoustic image production device 1 is illustrated in accordance with a first embodiment. FIG. 1 is an electrical block diagram of the photoacoustic image production device 1. FIG. 2 is a simplified perspective view of an ultrasonic probe 2. FIG. 3 is an electrical block diagram of an inside of the ultrasonic probe 2. FIG. 4 is a diagram illustrating a mechanism by which acoustic waves are generated by a photoacoustic effect. FIG. 5 is a flowchart of measurement processing. FIG. 6 is a timing chart of measurement operation.

As shown in FIG. 1, the photoacoustic image production device 1 in this embodiment comprises the ultrasonic probe 2 and a controller 3. The ultrasonic probe 2 is brought into contact with a test object A (such as a human body). The controller 3 controls the ultrasonic probe 2 and processes ultrasonic wave detection signals acquired by the ultrasonic probe 2.

The ultrasonic probe 2 has an ultrasonic probe unit 10 (e.g., the ultrasonic wave detector), a light source 20, and a light pulse driver 30 (e.g., a light source driver). As shown in FIG. 2, these are housed in an ultrasonic probe housing 2a. The light source 20 and the light pulse driver 30 are disposed near the ultrasonic probe unit 10. The ultrasonic probe 2 is connected to the controller 3 via a connecting wire 4. The connecting wire 4 includes a power supply line for supplying electrical power to the constituent elements in the ultrasonic probe 2, a signal line for performing communication, etc.

The ultrasonic probe unit 10 has an acoustic lens 11, an acoustic matching layer 12, a vibration element (piezoelectric element) 13, and a packing material 14. As shown in FIG. 2, the acoustic lens 11, the acoustic matching layer 12, the vibration element 13, and the packing material 14 are joined in that order.

The vibration element 13 receives ultrasonic waves from the outside, and produces an ultrasonic wave detection signal based on the reception of ultrasonic waves. The vibration element 13 includes a piezoelectric element. The vibration element 13 vibrates by receiving ultrasonic waves from the outside to generate a voltage signal (ultrasonic wave detection signal). The vibration element 13 can also vibrate by applying voltage to generate ultrasonic waves. Therefore, the vibration element 13 can both send and receive ultrasonic waves. The ultrasonic probe unit 10 in this embodiment is a linear ultrasonic probe. The vibration element 13 has numerous channels (such as 128 channels) disposed in a row in the width direction and at a specific spacing. Of course, the ultrasonic probe unit 10 is not limited to a linear type, and can instead be a convex type or a sector type. Thus, in the illustrated embodiment, the ultrasonic prove unit 10 produces the ultrasonic wave detection signal based on the received ultrasonic waves.

The packing material 14 is disposed on the rear face of the vibration element 13, and suppresses rearward propagation of ultrasonic waves. The acoustic matching layer 12 reduces the difference in acoustic impedance between the vibration element 13 and the test object A to allow more efficient transmission and reception of ultrasonic waves. The function of the acoustic lens 11 is to focus an ultrasonic wave beam and improve resolution.

The light source 20 is constituted by a plurality of light emitting diode (LED) chips. In this embodiment, the light source 20 includes an LED circuit in which 70 LED chips are connected in series. Consequently, the light source 20 can emit measurement light (e.g., light) having a specific level of light output. The measurement light is optical pulses having a pulse width of 150 nsec, and these optical pulses are intermittently emitted at repetition cycle.

In this embodiment, LED chips are used as the light emitting elements of the light source 20, but this is not the only option, and semiconductor laser chips or organic light emitting diodes can be used instead.

As shown in FIG. 3, the light pulse driver 30 comprises a booster circuit 31 and a driver (drive circuit) 32. The light pulse driver 30 supplies a drive voltage to the light source 20.

The booster circuit 31 is a DC-DC converter, and has a DC/AC converter (inverter) 31 a, a transformer 31 b, a rectification circuit 31 c, and a smoothing circuit 31 d. DC power is supplied from the controller 3 through the connecting wire 4 to the booster circuit 31, and the DC power supply voltage is boosted to a drive voltage of a specific voltage value (such as 210 VDC) in response to receiving a charging signal from the controller 3 that directs the start of a boost operation for producing drive voltage.

In this embodiment, the booster circuit 31 receives DC power supply voltage and performs the boost operation, but this is not the only option, and can instead receive AC power supply voltage and perform the boost operation.

With the booster circuit 31, a switching element 32a (a transistor or other such active element) is switched at the DC/AC converter 31 a to convert DC power supply voltage into AC voltage. The AC voltage is then boosted by the transformer 31 b, the boosted AC voltage is rectified by the rectification circuit 31 c and converted into DC voltage, and the boosted DC voltage is smoothed by the smoothing circuit 31 d. Consequently, at the smoothing circuit 31 d, a capacitor 32d is charged up to the drive voltage by the boosted DV voltage. In this embodiment, in a boost operation at specific repetition cycles during a measurement operation, the decreased charging voltage is boosted up to the drive voltage in approximately 150 µsec, and the booster circuit 31 stops the boost operation when the capacitor 32d has been fully charged.

A driver 33 is connected via the light source 20 to the booster circuit 31, and operates to apply drive voltage from the booster circuit 31 to the light source 20 for a specific emission period (such as a few dozen nanoseconds) in response to receiving an OPT-ON signal (optical pulse trigger signal) from the controller 3 for directing emission. Consequently, drive voltage is applied to the light source 20 for the specific emission period, and optical pulses having a pulse width corresponding to the emission period are emitted. Meanwhile, when drive voltage is supplied to the light source 20 for the emission period, the output voltage (charging voltage) of the booster circuit 31 decreases more than a specific drive voltage after the elapsed of the emission time. Therefore, the light pulse driver 30 operates the booster circuit 31 in response to a charging signal received from the controller 3, and restores the output voltage to the drive voltage.

As shown in FIG. 1, the controller 3 has a control component 40 (e.g., a controller of the present invention) and an image signal production component 50.

The control component 40 outputs an OPT-ON signal and a charging signal to the light pulse driver 30 in the ultrasonic probe 2 at specific repetition cycles, and thereby controls the operation of the ultrasonic probe 2.

In the illustrated embodiment, the control component 40 can include a microcomputer or a processor with a control program that controls the ultrasonic probe 2 as discussed below. The control component 40 can also include other conventional components such as an input interface circuit, an output interface circuit, and storage devices such as a ROM (Read Only Memory) device and a RAM (Random Access Memory) device. The internal RAM stores statuses of operational flags and various control data. The internal ROM stores the control programs for various operations. The control component 40 is capable of selectively controlling any of the components of the ultrasonic probe 2 or the controller 3 in accordance with the control program. It will be apparent to those skilled in the art from this disclosure that the precise structure and algorithms can be any combination of hardware and software that will carry out the functions of the present invention.

The image signal production component 50 has a reception circuit 52, an A/D converter 54, and an image composition component 56. The reception circuit 52 receives ultrasonic wave detection signals sent from the ultrasonic probe 2 through the connecting wire 4. The A/D converter 54 converts the ultrasonic wave detection signals received by the reception circuit 52 into digital signals. The image composition component 56 produces a sectional image signal (ultrasound image signal) based on the digital signal produced by the A/D converter 54. This ultrasound image signal is outputted to an image display component (display) 58, and a sectional image is displayed by the image display component 58. In the illustrated embodiment, the image composition component 56 can also include a microcomputer or a processor with a program that produces the sectional image signal. In the illustrated embodiment, the image signal production component 50 produces the ultra sound image based on the ultrasonic wave detection signal.

The photoacoustic effect produced by ultrasonic waves within the test object A by irradiation with optical pulses will now be described through reference to FIG. 4. We will consider a case in which an object B (such as a syringe) is located at a measurement depth L in the interior of the test object A. When an optical pulse Sa is emitted from the surface of the test object A toward the interior, the optical pulse Sa propagates at the speed of light C through the interior of the test object A, and reaches the object B in a certain propagation time Ta (= L/C). When the optical pulse Sa hits the object B, the internal molecules of the object absorb the optical energy of the optical pulse Sa and radiate heat. This radiant heat causes the object B to expand volumetrically, generating an acoustic wave (ultrasonic waves) Sb (photoacoustic effect). The acoustic wave Sb generated in this manner propagates at the speed of sound S through the interior of the test object A, and reaches the surface of the test object A in a certain propagation time Tb (= L/S). Thus, the reception period Tr from the start of emission of the optical pulse Sa until the reception of the acoustic wave Sb returning from the measurement depth L is complete is the sum of the propagation times Ta and Tb (Tr = Ta + Tb). However, since the speed of light C is far greater than the speed of sound S, the propagation time Ta in which the optical pulse Sa goes from the surface of the test object A to the object B can substantially be ignored. Thus, Tr ≈ Tb.

If the test object A is a human body, the speed of sound S can be assumed to be 1530 m/sec, for example.

The operation of the photoacoustic image production device 1 in this embodiment will now be described through reference to FIGS. 5 and 6.

When the user brings the ultrasonic probe 2 into contact with the surface of the test object A (the skin of the body) and begins measurement, the measurement switch of the controller 3 is turned on (step S1), whereupon the control component 40 commences the following processing.

First, the control component 40 sends a charging signal to the light pulse driver 30. This causes the booster circuit 31 to perform a boost operation. At this point, the capacitor 32d in the booster circuit 31 is charged from a discharged state to a fully charged state in about 10 seconds. The control component 40 then receives from the light pulse driver 30 a signal related to the charging voltage of the capacitor 32d of the smoothing circuit 31 d, and determines whether or not the charging voltage of the booster circuit 31 has been boosted up to the drive voltage (step S2).

If the charging voltage of the booster circuit 31 has not been boosted up to the drive voltage (No in step S2), then the control component 40 waits until the charging voltage is boosted up to the drive voltage. On the other hand, if the charging voltage has been boosted up to the drive voltage (Yes in step S2), then the control component 40 sends an OPT-ON signal to the light pulse driver 30 (step S3). As shown in FIG. 6, when the light pulse driver 30 receives the OPT-ON signal, the drive voltage is applied to the light source 20 for a specific emission period Topt (such as a few dozen nanoseconds), and the light source 20 emits an optical pulse having a pulse width of the emission period Topt toward the test object A.

This emission of the optical pulse generates an acoustic wave through the photoacoustic effect at various depths within the test object A. Also, when the drive voltage is supplied from the light pulse driver 30 to the light source 20, the voltage at both ends of the capacitor 32d that was charged up to the drive voltage by the booster circuit 31 in the light pulse driver 30 decreases below the drive voltage.

As shown in FIG. 6, the control component 40 waits for the elapse of a first reception period Tr1 from the output of the OPT-ON signal (that is, the start of the emission period Topt) (No in step S4) until the first reception period Tr1 elapses. This first reception period Tr1 is the period from the start of emission of the optical pulse until the ultrasonic probe unit 10 receives an acoustic wave (or an acoustic echo) generated at a specific measurement depth L within the test object A. In this embodiment, the measurement depth L (the maximum measureable depth) is set to 30 mm, and since as mentioned above Tr1 = L/C + L/S ≈ L/S, if S is 1530 m/sec, then the first reception period Tr1 is approximately 20 µsec.

Therefore, in this embodiment, since the measurement depth L is set to 30 mm, theoretically the ultrasonic probe unit 10 receives an acoustic wave generated at depths down to the measurement depth L (that is, from 0 to 30 mm). Consequently, the ultrasonic probe unit 10 produces an ultrasonic wave detection signal based on having received an acoustic wave, and sends this to the image signal production component 50. The image signal production component 50 produces a sectional image signal (ultrasound image signal) based on the ultrasonic wave detection signal received in the first reception period Tr1.

A sectional image of biological tissue of the test object A is displayed on the image display component 58 based on this ultrasound image signal. With the photoacoustic image production device 1 in this embodiment, an acoustic wave produced by the photoacoustic effect is made into an image. The syringe, which is made of metal, in the body is irradiated with optical pulses, and this produces acoustic waves based on the photoacoustic effect. Therefore, in this embodiment, a syringe or the like that has been injected into the biological tissue of the test object A can be displayed particularly clearly, and when giving an anesthetic injection, for example, it will be easy to tell that the tip of the syringe has reached the target location near the nerves in question.

Once the first reception period Tr1 has elapsed (Yes in step S4), the control component 40 sends a charging signal to the light pulse driver 30 (step S5). In this embodiment, the first reception period Tr1 is a charging prohibition period (or a charging stopped period). The control component 40 can send a charging signal simultaneously with the end of the first reception period Tr1, or can send a charging signal after a specific period (such as a few dozen microseconds) has elapsed.

The control component 40 then receives a signal related to the charging voltage of the capacitor 32d from the light pulse driver 30, and determines whether or not the charging voltage of the booster circuit 31 has reached the drive voltage (step S6). As shown in FIG. 6, during the charging period Tch in which the charging voltage has not yet reached the drive voltage (No in step S6), the control component 40 continues sending the charging signal. On the other hand, once the charging voltage reaches the drive voltage (Yes in step S6), the control component 40 halts the transmission of the charging signal. In this embodiment, the charging period Tch in which the charging signal is sent is approximately 150 µsec.

The control component 40 determines whether or not a specific measurement period since the start of measurement has ended (step S7), and if the measurement period has not ended (No in step S7), then the processing from step S3 to step S6 is repeated at a specific repetition cycle T (such as 1 msec to 100 msec), but if the measurement period has ended (Yes in step S7), the processing is ended.

Thus, in this embodiment, during the period in which the ultrasonic probe unit 10 is receiving ultrasonic waves (acoustic waves), that is, the first reception period Tr1 specified by the measurement depth L, the control component 40 controls the light pulse driver 30 so that the booster circuit 31 within the light pulse driver 30 will not perform its boost operation. In other words, the control component 40 controls the light pulse driver 30 so that operation of the booster circuit 31 within the light pulse driver 30 is stopped during the first reception period Tr1 in which the ultrasonic probe unit 10 receives ultrasonic waves produced in the test object A by the light emitted from the light source 20.

Consequently, electromagnetic noise attributable to the switching operation of the switching element 32a in the boost operation can be prevented from being mixed into the ultrasonic wave detection signal produced by the ultrasonic probe unit 10 and adversely affecting signal quality. This effectively avoids a decrease in sharpness or the occurrence of disturbance in the ultrasound image or the sectional image produced by the image signal production component 50.

In the illustrated embodiment, the photoacoustic image production device 1 is provided that comprises the light source 20 configured to emit light, the light pulse driver 30 (e.g., the light source driver) configured to cause the light source 20 to emit the light, the ultrasonic probe unit 10 (e.g., the ultrasonic wave detector) configured to receive an ultrasonic wave and produce an ultrasonic wave detection signal, the image signal production component 50 configured to produce an ultrasound image based on the ultrasonic wave detection signal, and the control component 40 (e.g., the controller) configured to control the light pulse driver 30 (e.g., the light source driver) to stop operation of the booster circuit 31 during the first reception period Tr1 in which the ultrasonic probe unit 10 (e.g., the ultrasonic wave detector) receives the ultrasonic wave produced by the light emitted from the light source 20.

In the illustrated embodiment, the first reception period Tr1 is a period from emission of the light by the light source 20 until reception of the ultrasonic wave produced at the specific measurement depth L in the test object A by the light.

In the illustrated embodiment, the light source 20 includes light emitting diodes, semiconductor laser chips or organic light emitting diodes that are serially connected to each other.

In the illustrated embodiment, the booster circuit 31 is a booster circuit for a light source driver.

In the illustrated embodiment, the light source 20 is disposed near the ultrasonic probe unit 10 (e.g., the ultrasonic wave detector).

In the illustrated embodiment, the light source 20 is disposed near the booster circuit 31.

In the illustrated embodiment, the light pulse driver 30 (e.g., the light source driver) is configured to drive the light source 20 with the specific drive voltage, and the control component 40 (e.g., the controller) is configured to control the light pulse driver 30 (e.g., the light source driver) to increase a charging voltage of the booster circuit 31 to the drive voltage (e.g., the charging period Tch) after emission of the light by the light source 20 (after the emission period Topt).

In the illustrated embodiment, the control component (e.g., the controller) is configured to control the light pulse driver 30 (e.g., the light source driver) to increase the charging voltage of the booster circuit 31 to the drive voltage (e.g., the charging period Tch) after (or in response to) elapsing the first reception period Tr1.

In the illustrated embodiment, the light source 20, the light pulse driver 30 (e.g., the light source driver) and the ultrasonic probe unit 10 (e.g., the ultrasonic wave detector) are disposed within the ultrasonic probe housing 2a (e.g., the housing).

### SECOND EMBODIMENT

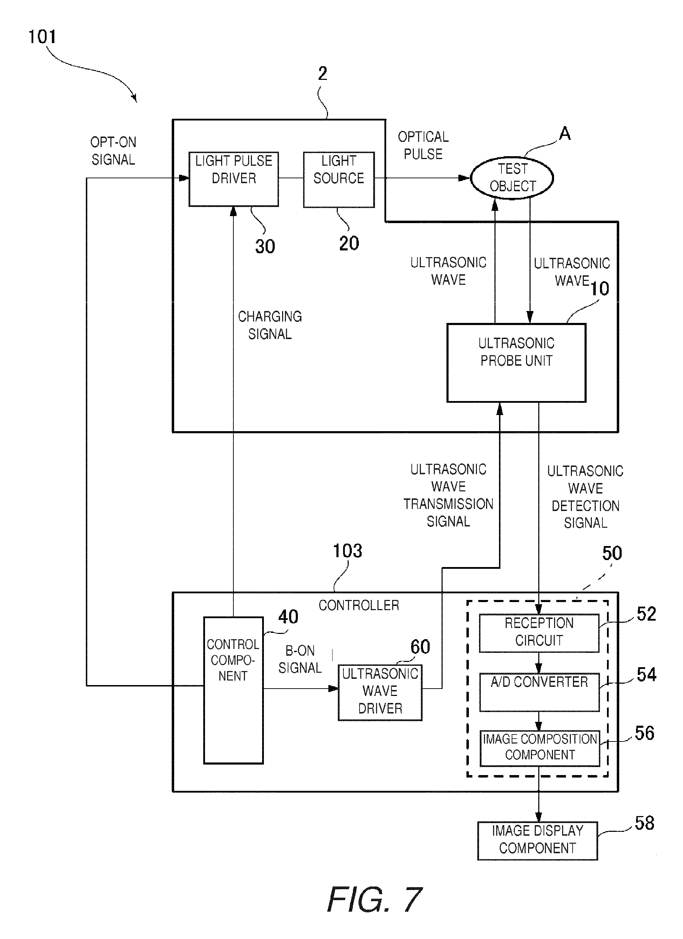

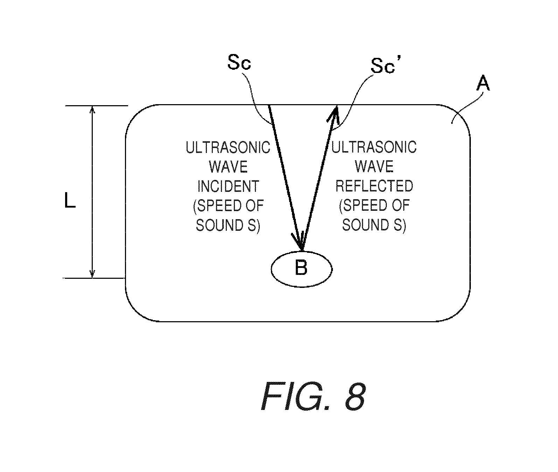

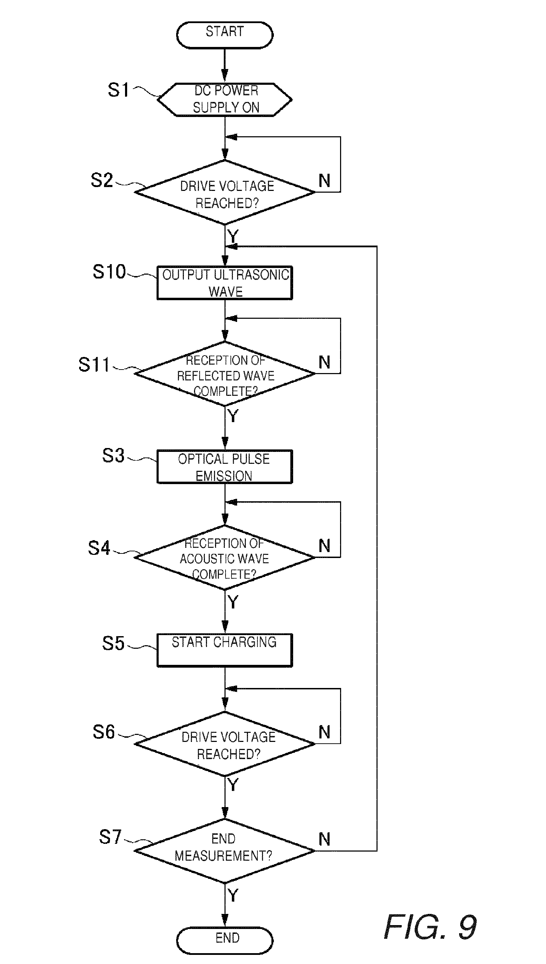

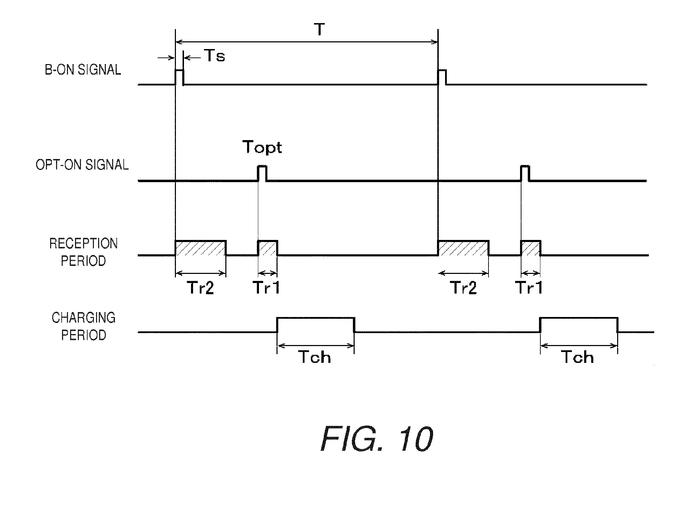

Referring now to FIGS. 7 to 10, a photoacoustic image production device 101 in accordance with a second embodiment will now be explained. In view of the similarity between the first and second embodiments, the parts of the second embodiment that are identical to the parts of the first embodiment will be given the same reference numerals as the parts of the first embodiment. Moreover, the descriptions of the parts of the second embodiment that are identical to the parts of the first embodiment may be omitted for the sake of brevity. FIG. 7 is an electrical block diagram of the photoacoustic image production device 101. FIG. 8 is a diagram illustrating a mechanism by which reflected waves are generated. FIG. 9 is a flowchart of measurement processing. FIG. 10 is a timing chart of measurement operation.

As shown in FIG. 7, the photoacoustic image production device 101 in this embodiment comprises an ultrasonic probe 2 and a controller 103.

In this embodiment, an ultrasonic probe unit 10 in the ultrasonic probe 2 is configured not only to receive ultrasonic waves, but also to transmit ultrasonic waves. Therefore, the ultrasonic probe unit 10 receives an ultrasonic wave transmission signal having a specific drive voltage (such as a positive or negative voltage of 50 to 100 V) from the controller 103, and in response to this, generates ultrasonic waves or ultrasonic pulses by vibrating a vibration element 13 during the period in which the ultrasonic wave transmission signal is being received.

The controller 103 has an ultrasonic wave driver 60 (e.g., an ultrasonic wave driving circuit) in addition to a control component 40 (e.g., a controller of the present invention) and an image signal production component 50.

The control component 40 sends the ultrasonic wave driver 60 a B-ON signal (ultrasonic wave signal trigger signal) for instructing to generate ultrasonic waves, at a specific repetition cycle. Upon receiving the B-ON signal from the control component 40, the ultrasonic wave driver 60 sends an ultrasonic wave transmission signal to the ultrasonic probe unit 10 in response to this B-ON signal. Consequently, the controller 103 controls the ultrasonic probe unit 10 of the ultrasonic probe 2.

In addition to the ultrasonic wave detection signal based on acoustic waves received as a result of the emission of optical pulses, the image signal production component 50 also performs similar signal processing on an ultrasonic wave detection signal based on reflected waves of ultrasonic wave pulses outputted from the ultrasonic probe unit 10, and produces an ultrasound image signal. Here, the ultrasound image signal based on the reception of acoustic waves can be superposed with the ultrasound image signal based on the reception of reflected waves. Consequently, a sectional image in which these two signals are superposed is displayed on an image display component 58. Therefore, in this embodiment, an image of a metal needle or the like that is easily detected by acoustic waves is superposed with an image of biological tissue that is easily detected by reflected waves, which allows the position of the metal needle, etc., in the body to be identified more clearly.

A reflected wave received when an outputted ultrasonic wave is reflected within a test object A will now be described through reference to FIG. 8. We will consider a case in which an object B (such as a blood vessel, nerve tissue, etc.) is located at a measurement depth L in the interior of the test object A. When a pulse-form ultrasonic wave Sc is outputted from the surface of the test object A toward the interior, the ultrasonic wave Sc propagates at the speed of sound S through the interior of the test object A, and reaches an object B in a certain propagation time Tb (= L/S). Since the object B is a medium with different acoustic properties from those of the surrounding tissue, the ultrasonic wave Sc is reflected by the object B. The reflected wave Sc' thus reflected propagates at the speed of sound S through the interior of the test object A, and reaches the surface of the test object A in the same propagation time Tb. Thus, the reception period Tr from the start of emission of the ultrasonic wave Sc until the reception of the reflected wave Sc' reflected at the measurement depth L is complete is a length of time that is two times the propagation time Tb (Tr = Tb x 2).

The operation of the photoacoustic image production device 101 in this embodiment will now be described through reference to FIGS. 9 and 10.

In the measurement processing flowchart in FIG. 9, steps S10 and S11 are added between steps S2 and S3 in the flowchart of FIG. 5.

When the user brings the ultrasonic probe 2 into contact with the surface of the test object A (the skin of the body) and begins measurement, the measurement switch of the controller 103 is turned on (step S1), whereupon the control component 40 commences the following processing.

First, by the processing in step S1, the control component 40 receives a signal related to charging voltage from a light pulse driver 30, and determines whether or not the charging voltage has been boosted up to the drive voltage (step S2).

The control component 40 waits for the charging voltage of a booster circuit 31 to be boosted up to the drive voltage (No in step S2), and once the charging voltage is boosted up to the drive voltage (Yes in step S2), the control component 40 sends the ultrasonic wave driver 60 a B-ON signal (step S10), as shown in FIG. 10. Consequently, drive voltage is sent from the ultrasonic wave driver 60 to the vibration element 13 of the ultrasonic probe unit 10 for a specific output period Ts (such as a few dozen nanoseconds), and an ultrasonic wave pulse is outputted from the ultrasonic probe unit 10 toward the inside of the test object A. This ultrasonic wave pulse is reflected at various depths within the test object A, and the reflected waves are returned successively to the ultrasonic probe unit 10.

As shown in FIG. 10, the control component 40 waits for the elapse of a second reception period Tr2 from the output of the B-ON signal (that is, the start of the output period Ts) (No in step S11). This second reception period Tr2 is the period from the start of output of the ultrasonic wave pulse until the ultrasonic probe unit 10 receives a reflected wave at a specific measurement depth L within the test object A. In this embodiment, as discussed above, the measurement depth L is set to 30 mm, and since Tr2 = L/S x 2, if S is 1530 m/sec, then the second reception period Tr2 is approximately 40 µsec.

Therefore, in this embodiment, since the measurement depth L is set to 30 mm, theoretically the ultrasonic probe unit 10 receives a reflected wave reflected at depths down to the measurement depth L (that is, from 0 to 30 mm). Consequently, the ultrasonic probe unit 10 produces an ultrasonic wave detection signal based on having received a reflected wave, and sends this to the image signal production component 50.

Once the second reception period Tr2 has elapsed (Yes in step S11), the control component 40 sends an OPT-ON signal to the light pulse driver 30 (step S3). The OPT-ON signal is transmitted after a specific length of time (such as a few dozen microseconds) has further elapsed since the elapse of the second reception period Tr2, but the configuration can instead be such that the OPT-ON signal is transmitted simultaneously with the elapse of the second reception period Tr2.

As shown in FIG. 10, when the light pulse driver 30 receives the OPT-ON signal, and drives the light source 20 to emit an optical pulse with a specific emission period Topt (such as a few dozen nanoseconds).

The control component 40 waits for the elapse of the first reception period Tr1 from the output of the OPT-ON signal (that is, the start of the emission period Topt) (No in step S4) until the first reception period Tr1 elapses. This first reception period Tr1 is the period from the start of emission of the optical pulse until the ultrasonic probe unit 10 receives an acoustic wave (or an acoustic echo) generated at a specific measurement depth L within the test object A. In this embodiment, since the measurement depth L is set to 30 mm as mentioned above, the first reception period Tr1 is approximately 20 µsec.

Therefore, in this embodiment, the ultrasonic probe unit 10 receives an acoustic wave generated at depths down to the measurement depth L, produces an ultrasonic wave detection signal based on this acoustic wave, and sends this to the image signal production component 50.

The image signal production component 50 produces an ultrasound image signal by combining an ultrasonic wave detection signal based on the reception of an ultrasonic wave in the second reception period Tr2 with an ultrasonic wave detection signal based on the reception of an ultrasonic wave in the first reception period Tr1. In the second reception period Tr2, a reflected wave is received that is produced by the difference in acoustic characteristics in the biological tissue of the test object A, and the ultrasonic wave detection signal in this period is suited to clearly indicating the biological tissue of the test object A. Meanwhile, in the first reception period Tr1, an acoustic wave is received that is produced by the photoacoustic effect within the test object A, and the ultrasonic wave detection signal in this period is suited to clearly indicating a syringe or the like in the body.

Thus, with the photoacoustic image production device 101 in this embodiment, ultrasonic wave detection signals in the first and second reception periods Tr1 and Tr2 are combined, which makes it possible for a syringe or the like in biological tissue to be displayed more clearly.

On the other hand, if the first reception period Tr1 has elapsed (Yes in step S4), the control component 40 sends a charging signal to the light pulse driver 30 (step S5). In this embodiment, the first reception period Tr1 and the second reception period Tr2 are charging prohibited periods (or charging stopped periods). The control component 40 can send the charging signal simultaneously with the end of the first reception period Tr1, or can send the charging signal after a specific period (such as a few dozen microseconds) has elapsed.

The control component 40 then receives from the light pulse driver 30 a signal related to the charging voltage of the capacitor 32d, and determines whether or not the charging voltage of the booster circuit 31 has reached the drive voltage (step S6). As shown in FIG. 10, during the charging period Tch in which the charging voltage has not yet reached the drive voltage (No in step S6), the control component 40 continues to transmit the charging signal. When the charging voltage reaches the drive voltage (Yes in step S6), the control component 40 halts the transmission of the charging signal. In this embodiment, the charging period Tch in which the charging signal is transmitted is approximately 150 µsec.

If the measurement period has not yet ended (No in step S7), then the control component 40 repeats the processing from steps S10 to S6 at a specific repetition cycle (such as 1 msec to 100 msec), but if the measurement period has ended (Yes in step S7), then the processing is ended.

Thus, in this embodiment, during the period when the ultrasonic probe unit 10 is receiving ultrasonic waves (reflected waves and acoustic waves), that is, during the first reception period Tr1 and the second reception period Tr2 specified by the measurement depth L, the control component 40 controls the booster circuit 31 of the light pulse driver 30 so that no boost operation is executed by the booster circuit 31. Consequently, electromagnetic noise attributable to the switching operation of a switching element 32a in the boost operation can be prevented from being mixed into the ultrasonic wave detection signal produced by the ultrasonic probe unit 10 and adversely affecting signal quality. This effectively avoids a decrease in sharpness or the occurrence of disturbance in the ultrasound image signal produced by the image signal production component 50.

In the illustrated embodiment, the photoacoustic image production device 101 further comprises the ultrasonic probe unit 10 (e.g., the ultrasonic wave generator) configured to output an ultrasonic wave in the test object A, the ultrasonic probe unit 10 (e.g., the ultrasonic wave detector) being configured to receive a reflected wave produced when the ultrasonic wave outputted from the ultrasonic probe unit 10 (e.g., the ultrasonic wave generator) is reflected inside the test object A, and configured to produce an ultrasonic wave detection signal based on the received reflected wave.

In the illustrated embodiment, the control component 40 (e.g., the controller) is configured to control output of the ultrasonic wave by the ultrasonic probe unit 10 (e.g., the ultrasonic wave generator), and configured to control the light pulse driver 30 (e.g., the light source driver) to stop the operation of the booster circuit 31 during the second reception period Tr2 in which the ultrasonic probe unit 10 (e.g., the ultrasonic wave detector) receives the reflected wave of the ultrasonic wave outputted from the ultrasonic probe unit 10 (e.g., the ultrasonic wave generator).

In the illustrated embodiment, the second reception period Tr2 is a period from the output of ultrasonic wave by the ultrasonic probe unit 10 (e.g., the ultrasonic wave generator) until reception of the reflected wave produced by reflecting the ultrasonic wave at the specific measurement depth L within the test object A.

In the illustrated embodiment, the control component 40 (e.g., the controller) is configured to control the light pulse driver 30 (e.g., the light source driver) to drive the booster circuit 31 for the charging period (e.g., the specific drive period) at the specific repetition cycle T and to emit the light from the light source 20 for the specific emission period Topt, and configured to control the ultrasonic probe unit 10 (e.g., the ultrasonic wave generator) to output the ultrasonic wave from the ultrasonic probe unit 10 (e.g., the ultrasonic wave generator) for the specific output period Ts, the specific repetition cycle T is at least a period obtained by adding the first reception period Tr1, the second reception period Tr2, and the drive period Tch of the booster circuit 31.

In the illustrated embodiment, the ultrasonic probe unit 10 (e.g., the ultrasonic wave detector and the ultrasonic wave generator) commonly includes the vibration element 13 (e.g., the piezoelectric element).

In the illustrated embodiment, the light pulse driver 30 (e.g., the light source driver) is configured to drive the light source 20 with the specific drive voltage, and the control component 40 (e.g., the controller) is configured to control the light pulse driver 30 (e.g., the light source driver) to increase a charging voltage of the booster circuit 31 to the drive voltage after the output of the ultrasonic wave by the ultrasonic probe unit 10 (e.g., the ultrasonic wave generator).

In the illustrated embodiment, the control component 40 (e.g., the controller) is configured to control the light pulse driver 30 (e.g., the light source driver) to increase the charging voltage of the booster circuit 31 to the drive voltage after (or in response to) elapsing the second reception period Tr2.

In the illustrated embodiment, the control component 40 (e.g., the controller) is configured to control the light pulse driver 30 (e.g., the light source driver) to increase the charging voltage of the booster circuit 31 to the drive voltage after (or in response to) elapsing both the first and second reception periods Tr1 and Tr2.

In the illustrated embodiment, the control component 40 (e.g., the controller) is configured to control the light pulse driver 30 (e.g., the light source driver) to increase a charging voltage of the booster circuit 31 to the drive voltage after the output of the ultrasonic wave by the ultrasonic probe unit 10 (e.g., the ultrasonic wave generator) and emission of the light by the light source 20.

In the illustrated embodiment, the image signal production component 50 is configured to produce the ultrasound image by combining the ultrasonic wave detection signal based on the received reflected wave in response to output of the ultrasonic wave by the ultrasonic probe unit 10 (e.g., the ultrasonic wave generator) with the ultrasonic wave detection signal based on the ultrasonic wave in response to emission of the light by the light source 20.

The present invention is not limited to or by the above embodiments, and can be modified as follows.

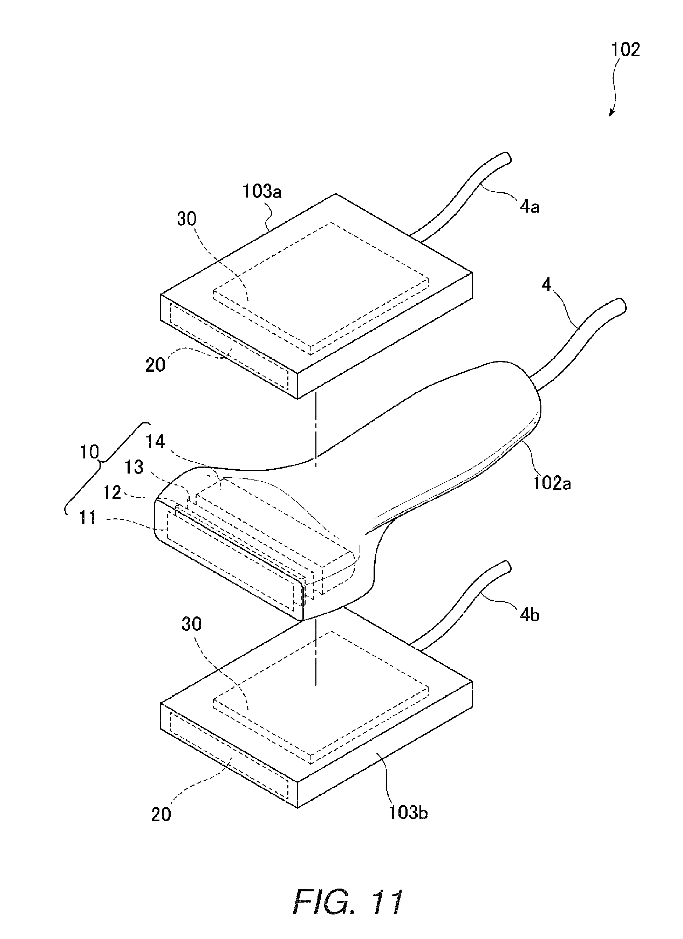

The ultrasonic probe 2 shown in FIG. 2 is such that the light source 20 and the light pulse driver 30 are housed along with the ultrasonic probe unit 10 inside the ultrasonic probe housing 2a. However, this is not the only option. An ultrasonic probe 102 can be configured as shown in the exploded perspective view of FIG. 11. In FIG. 11, the ultrasonic probe 102 is configured separately from illumination devices 103a and 103b, and the illumination devices 103a and 103b are removably or permanently attached on both side faces in the width direction of the ultrasonic probe 102.

The ultrasonic probe 102 houses the ultrasonic probe unit 10 inside of an ultrasonic probe housing 102a. Meanwhile, the illumination devices 103a and 103b house light sources 20 and light pulse drivers 30 in their respective housings, and are connected to the controller 3 via connecting wires 4a and 4b, respectively.

Even when using the illumination devices 103a and 103b that are thus separate from the ultrasonic probe 102, these illumination devices 103a and 103b are preferably disposed near the ultrasonic probe unit 10 of the ultrasonic probe 102. Therefore, with this arrangement, there can be a risk that the image quality of the ultrasound image signal will be adversely affected by electromagnetic noise produced by the boost operation at the illumination devices 103a and 103b without taking any measures. However, deterioration of the image quality can be effectively avoided by setting the charging prohibited period as discussed in the above-mentioned illustrated embodiments.

In the illustrated example, the light source 20 and the light pulse driver 30 (e.g., the light source driver) are disposed within a housing that is separate from a housing of the ultrasonic probe unit 10 (e.g., the ultrasonic wave detector).

Also, in the second embodiment above, in the repetition cycle T, first an ultrasonic wave pulse is outputted and the second reception period Tr2 is set, and then an optical pulse is emitted and the first reception period Tr1 is set, after which the charging period Tch is set. However, if the charging period Tch does not temporally overlap the first reception period Tr1 and the second reception period Tr2, the charging period Tch can be set to any period within the repetition cycle T. For instance, the first reception period Tr1 can be set first, then the second reception period Tr2, and finally the charging period Tch, or the first reception period Tr1 can be set first, then the second reception period Tr2, but the charging period Tch can be divided up into between these periods and after the second reception period Tr2.

One aspect of the present application is a photoacoustic image production device that comprises a light source that shines light into a test object, a light source driver that supplies a specific drive voltage to cause the light source to emit light, an ultrasonic wave detector that receives ultrasonic waves and produces an ultrasonic wave detection signal based on the received ultrasonic waves, an image signal production component that produces an ultrasound image of a test object based on the ultrasonic wave detection signal produced by the ultrasonic wave detector, and a controller that controls the light source driver, wherein the controller controls the light source driver so that operation of a booster circuit within the booster circuit within the light source driver is stopped during a first reception period in which the ultrasonic wave detector receives ultrasonic waves produced in the test object by light emitted from the light source.

With the present application configured in this way, when a specific drive voltage produced by the booster circuit within the light source driver is supplied to the light source, light from the light source is shined toward the inside of the test object, a photoacoustic effect within the test object produces ultrasonic waves (acoustic waves), an ultrasonic wave detection signal is produced by the ultrasonic wave detector based on these acoustic waves, and a photoacoustic image (ultrasound image) of the test object is produced by the image signal production component based on this ultrasonic wave detection signal. With the present application, when drive voltage is supplied, the output voltage of the light source driver is lowered, so the booster circuit can be operated in response to the supply of drive voltage to the light source, and the output voltage can be restored to the drive voltage.

However, the operation of this booster circuit is accompanied by the switching operation of the switching element, electromagnetic noise can be produced as a result of this operation, and there is a risk that this electromagnetic noise will be mixed into the ultrasonic wave detection signal produced by the ultrasonic wave detector. This admixture of electromagnetic noise lowers the quality of the ultrasound image produced by the image signal production component.

When a solid state laser device (a large device) is used as the light source, the solid state laser device is configured separately from the ultrasonic probe, so even if electromagnetic noise should occur, a shielding structure can be easily added, which means that the adverse effect of the electromagnetic noise can be mitigated. However, when an LED chip or a semiconductor laser chip is used as a light source, a new configuration is required that will reduce the effect of electromagnetic noise. In particular, since an LED chip or the like is small, a configuration is possible in which the light source is disposed in or near the ultrasonic probe housing that houses the ultrasonic wave detector, but because the light source is near the ultrasonic wave detector, the effect of any electromagnetic noise ends up being more pronounced. However, if a physical shielding structure is provided to the ultrasonic probe, the ultrasonic probe increases in size and weight and becomes more unwieldy to use.

In view of this, with the present application, the configuration is such that the boost operation by the booster circuit that accompanies the switching operation of the switching element is stopped during the first reception period in which light is emitted from the light source and the acoustic waves produced by this light are received by the ultrasonic wave detector. Consequently, with the present application, rather than providing the ultrasonic probe with a structure that physically blocks electromagnetic noise, the control configuration is designed to prevent electromagnetic noise from being mixed into the ultrasonic wave detection signal, and deterioration in the quality of the ultrasound image can be effectively suppressed.

With the present application, it is preferable if the first reception period is the period from the emission of light by the light source until the reception of ultrasonic waves produced at a specific measurement depth in the test object by this light.

With the present application configured in this way, ultrasonic waves produced by the photoacoustic effect at a specific measurement depth of the test object, or deeper, during the first reception period. This allows measurement to be performed at the specific measurement depth, and suppresses the effect of electromagnetic noise.

With the present application, it is preferable if the photoacoustic image production device further comprises an ultrasonic wave generator that outputs ultrasonic waves in a test object, wherein the ultrasonic wave detector receives reflected waves produced when the ultrasonic waves outputted from the ultrasonic wave generator are reflected inside the test object, and produces an ultrasonic wave detection signal based on the received reflected waves, and the controller controls the output of ultrasonic waves produced by the ultrasonic wave generator, and controls the light source driver so as to stop the operation of the booster circuit also during a second reception period in which the ultrasonic wave detector receives the reflected waves of the ultrasonic waves outputted from the ultrasonic wave generator.

With the present application configured in this way, measurement-use ultrasonic waves can be outputted from the ultrasonic wave generator toward the inside of the test object, and the reflected waves produced when these ultrasonic waves are reflected in the interior of the test object can be received by the ultrasonic wave detector. Consequently, an ultrasound image produced by the output of ultrasonic waves can be acquired in addition to an ultrasound image produced by the emission of light. However, if the switching operation of the switching element is performed in the booster circuit during the second reception period in which the reflected waves of the ultrasonic waves are received by the ultrasonic wave detector, the electromagnetic noise will end up being added to the ultrasonic wave detection signal. In view of this, in order to avoid the adverse effect of this electromagnetic noise, in the present application the configuration is such that the switching operation of the switching element in the booster circuit is stopped not only in the first reception period, but also in the second reception period. Consequently, with the present application, electromagnetic noise is prevented from being mixed into the ultrasonic wave detection signal also while reflected waves corresponding to measurement-use ultrasonic waves are being received, and deterioration in the quality of the ultrasound image can be effectively suppressed.

With the present application, it is preferable if the second reception period is the period from the output of ultrasonic waves by the ultrasonic wave generator until the reception of reflected waves produced when these ultrasonic waves are reflected at a specific measurement depth within the test object.

With the present application configured in this way, during the second reception period the reflected waves (ultrasonic waves) produced by reflection at a specific measurement depth of the test object, or deeper. Consequently, measurement at the specific measurement depth can be ensured while the effect of electromagnetic noise can be suppressed.

With the present application, it is preferable if the controller controls the light source driver so that the booster circuit is driven for a specific drive period at specific repetition cycles and light is emitted from the light source for a specific emission period, and controls the ultrasonic wave generator so that ultrasonic waves are outputted from the ultrasonic wave generator for a specific output period, and the specific repetition cycle is at least a period obtained by adding the first reception period, the second reception period, and the drive period of the booster circuit.

With the present application configured in this way, an ultrasonic wave detection signal based on the emission of light, and an ultrasonic wave detection signal based on the output of ultrasonic waves can be acquired at specific repetition cycles. The specific repetition cycle is at least a period obtained by adding the first reception period, the second reception period, and the drive period of the booster circuit, and within the repetition cycle the boost operation is performed during a period outside the first reception period and the second reception period, so no electromagnetic noise will be mixed into either of the ultrasonic wave detection signals, and deterioration in the image quality can be suppressed.

With the present application, it is preferable if the ultrasonic wave detector includes a piezoelectric element and also serves as the ultrasonic wave generator.

With the present application configured in this way, the ultrasonic wave detector can use a piezoelectric element to convert ultrasonic waves into electrical signals, while the ultrasonic wave generator can use a piezoelectric element to convert electrical signals into ultrasonic waves.

With the photoacoustic image production device of the present application, electromagnetic noise produced by the switching operation of the switching element in the booster circuit that produces the drive voltage of the light source is prevented from being mixed into the ultrasonic wave detection signal, and deterioration in the produced ultrasound image caused by electromagnetic noise can be prevented.

In understanding the scope of the present invention, the term "comprising" and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having" and their derivatives. Also, the terms "part," "section," "portion," "member" or "element" when used in the singular can have the dual meaning of a single part or a plurality of parts unless otherwise stated.

While only selected embodiments have been chosen to illustrate the present invention, it will be apparent to those skilled in the art from this disclosure that various changes and modifications can be made herein without departing from the scope of the invention as defined in the appended claims. For example, unless specifically stated otherwise, the size, shape, location or orientation of the various components can be changed as needed and/or desired so long as the changes do not substantially affect their intended function. Unless specifically stated otherwise, components that are shown directly connected or contacting each other can have intermediate structures disposed between them so long as the changes do not substantially affect their intended function. The functions of one element can be performed by two, and vice versa unless specifically stated otherwise. The structures and functions of one embodiment can be adopted in another embodiment. It is not necessary for all advantages to be present in a particular embodiment at the same time. Every feature which is unique from the prior art, alone or in combination with other features, also should be considered a separate description of further inventions by the applicant, including the structural and/or functional concepts embodied by such feature(s). Thus, the foregoing descriptions of the embodiments according to the present invention are provided for illustration only, and not for the purpose of limiting the invention as defined by the appended claims and their equivalents.

## Claims

1. A photoacoustic image production device comprising:
a light source configured to emit light;
a light source driver configured to cause the light source to emit the light;
an ultrasonic wave detector configured to receive an ultrasonic wave and produce an ultrasonic wave detection signal;
an image signal production component configured to produce an ultrasound image based on the ultrasonic wave detection signal; and
a controller configured to control the light source driver to stop operation of a booster circuit during a first reception period in which the ultrasonic wave detector receives the ultrasonic wave produced by the light emitted from the light source.

2. The photoacoustic image production device according to claim 1,
wherein
the first reception period is a period from emission of the light by the light source until reception of the ultrasonic wave produced at a specific measurement depth in a test object by the light.

3. The photoacoustic image production device according to claim 1 or 2, wherein
the light source includes light emitting diodes, semiconductor laser chips or organic light emitting diodes that are serially connected to each other.

4. The photoacoustic image production device according to any one of claims 1 to 3, wherein
the booster circuit is a booster circuit for a light source driver.

5. The photoacoustic image production device according to any one of claims 1 to 4, further comprising
an ultrasonic wave generator configured to output an ultrasonic wave in a test object,
the ultrasonic wave detector being configured to receive a reflected wave produced when the ultrasonic wave outputted from the ultrasonic wave generator is reflected inside the test object, and configured to produce an ultrasonic wave detection signal based on the received reflected wave.

6. The photoacoustic image production device according to claim 5,
wherein
the controller is configured to control output of the ultrasonic wave by the ultrasonic wave generator, and configured to control the light source driver to stop the operation of the booster circuit during a second reception period in which the ultrasonic wave detector receives the reflected wave of the ultrasonic wave outputted from the ultrasonic wave generator.

7. The photoacoustic image production device according to claim 6,
wherein
the second reception period is a period from the output of ultrasonic wave by the ultrasonic wave generator until reception of the reflected wave produced by reflecting the ultrasonic wave at a specific measurement depth within the test object.

8. The photoacoustic image production device according to claim 6 or 7, wherein
the controller is configured to control the light source driver to drive the booster circuit for a specific drive period at specific repetition cycle and to emit the light from the light source for a specific emission period, and configured to control the ultrasonic wave generator to output the ultrasonic wave from the ultrasonic wave generator for a specific output period,
the specific repetition cycle is at least a period obtained by adding the first reception period, the second reception period, and the drive period of the booster circuit.

9. The photoacoustic image production device according to any one of claims 6 to 8, wherein
the controller is configured to control the light source driver to increase the charging voltage of the booster circuit to the drive voltage after elapsing the second reception period.

10. The photoacoustic image production device according to any one of claims 5 to 9, wherein
the light source driver is configured to drive the light source with a specific drive voltage, and
the controller is configured to control the light source driver to increase a charging voltage of the booster circuit to the drive voltage after the output of the ultrasonic wave by the ultrasonic wave generator.

11. The photoacoustic image production device according to any one of claims 5 to 10, wherein
the ultrasonic wave detector and the ultrasonic wave generator commonly include a piezoelectric element.

12. The photoacoustic image production device according to any one of claims 1 to 11, wherein
the light source is disposed near the ultrasonic wave detector.

13. The photoacoustic image production device according to any one of claims 1 to 12, wherein
the light source is disposed near the booster circuit.

14. The photoacoustic image production device according to any one of claims 1 to 13, wherein
the light source driver is configured to drive the light source with a specific drive voltage, and
the controller is configured to control the light source driver to increase a charging voltage of the booster circuit to the drive voltage after emission of the light by the light source.

15. The photoacoustic image production device according to any one of claims 1 to 14, wherein
the controller is configured to control the light source driver to increase the charging voltage of the booster circuit to the drive voltage after elapsing the first reception period.
